# EUROPEAN PATENT SPECIFICATION

(11) **EP 4 251 248 B1**
(45) Date of publication and mention of the grant of the patent: **21.08.2024**
(21) Application number: 21827818.2
(22) Date of filing: 25.11.2021
(51) Int. Cl.: A61M 16/16, A62B 9/00, B05B 7/00

(54) **HUMIDIFIER DEVICE OF DRY GASEOUS FLUID**
BEFEUCHTER FÜR TROCKENES GASFÖRMIGES FLUID
DISPOSITIF HUMIDIFICATEUR DE FLUIDE GAZEUX SEC

(30) Priority: 27.11.2020 IT 202000028811
(43) Date of publication of application: 04.10.2023
(73) Proprietor: Quantimek di Bertoncello Claudio, 31030 Borso del Grappa, (TV) (IT)
(72) Inventor: BERTONCELLO, Claudio, 31030 Borso del Grappa, Treviso (IT)
(74) Representative: Busana, Omar
(86) International application number: PCT/IB2021/060957
(87) International publication number: WO 2022/112983

(56) References cited:
- WO-A1-03/061746
- CN-A- 108 543 189
- CN-U- 207 898 753
- FR-A1- 2 409 054
- FR-A5- 2 045 205
- JP-A- H10 137 339
- US-A- 3 881 480

## Description

### FIELD OF APPLICATION

The present invention relates to a humidifier device of a gaseous fluid. In particular, the present invention relates to a humidifier device of a gaseous fluid which may be used in a self-contained breathing apparatus.

### PRIOR ART

In this discussion, reference will be made to a particular application of the humidifier device of a gaseous fluid, that is, to its application in a self-contained breathing apparatus. This reference is obviously intended for explanatory and non-limiting purposes only of the applications in which the device may be used, as will be discussed in detail below.

As is known, there are numerous applications in which a humidifier device of a gaseous fluid is used, to increase the humidity content of a fluid which may initially be dry or substantially dry.

For example, the compressed air contained inside the cylinders of self-contained breathing apparatus has a very low humidity content, and when it is delivered through the mouthpiece, it may cause annoying irritation to the user's respiratory tract.

The devices of the known type, although widely used and appreciated, are not free from drawbacks.

For example, many of the known devices are complex devices which, by their nature, are subject to malfunctions; if the malfunction occurs in an apparatus used underwater or in an apparatus used in emergency situations, it may cause serious problems for the user's health.

Furthermore, the simplest devices, designed and built to avoid certain problems linked to possible malfunctions, for example, do not allow an adjustment of the degree of humidity to be supplied to the dry gaseous fluid.

The documents JP H10 137339 A, FR 2 409 054 A1, CN 207 898 753 U, FR 2 045 205 A5, US 3 881 480 A, CN 108 543 189 A and WO 03/061746 A1 disclose some prior art humidifier devices. The document JP H10 137339 A, for example, discloses a humidifier device which includes a container with an inlet into which concentrated oxygen is introduced. The container stores water for humidification purposes. The oxygen is humidified with the sprayed water in a venturi tube provided inside the container. A water intake pipe supplies the water into the venturi tube. An outlet discharges the humidified oxygen into the supply unit of an oxygen condenser.

### DISCLOSURE OF THE INVENTION

The need to solve the drawbacks and limitations mentioned with reference to the prior art is therefore felt.

Therefore, the need is felt to provide a humidifier device which may be installed in a self-contained breathing apparatus and which, due to its technical conformation, is less subject to malfunctions than the humidifier devices of the prior art.

Furthermore, the need is felt for a device which is not very susceptible to malfunctions, and which at the same time allows the degree of humidity of a dry gaseous fluid to be adjusted according to the specific needs of the user.

These requirements are met, at least partially, by a humidifier device according to claim 1.

### DESCRIPTION OF THE DRAWINGS

Further features and advantages of the present invention will become more apparent from the following description of preferred and non-limiting embodiments thereof, in which:
- Figure 1 schematically shows a sectional view of a humidifier device according to a possible embodiment of the present invention;

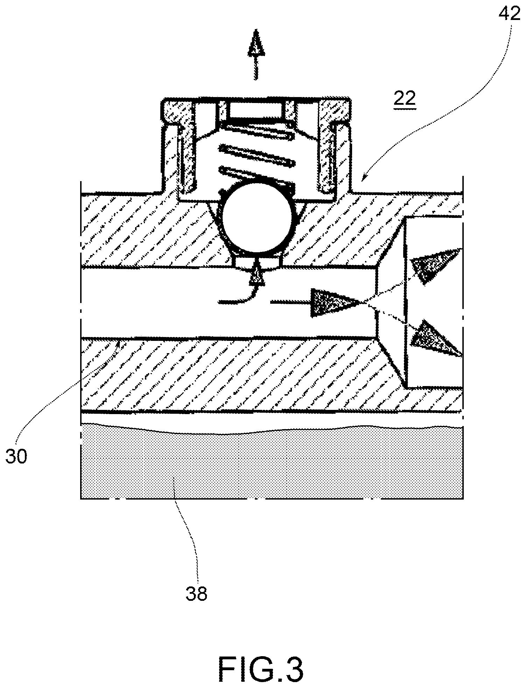

- Figures 2 and 3 schematically show two views of enlarged details of Figure 1; and
- Figure 4 schematically shows a possible alternative embodiment of a component of a humidifier device according to a possible embodiment of the present invention.

Elements or parts of elements common to the embodiments described hereinafter will be indicated with the same reference numerals.

### DETAILED DESCRIPTION

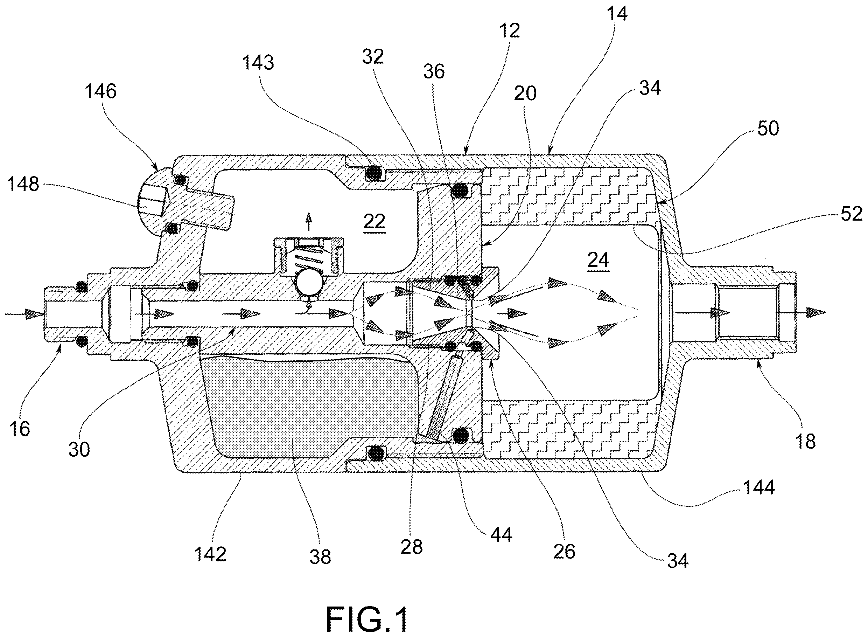

In Figure 1, with the reference numeral 12 it is generally indicated a humidifier device of dry gaseous fluid according to the present invention.

The device 12 comprises a body 14 provided with an inlet connection 16 and an outlet connection 18, the inside of which is provided with a separator 20 adapted to divide the inner volume of said body into two chambers 22, 24: a first compensation chamber 22 provided near the inlet connection 16; and a second expansion and nebulization chamber 24 provided near the outlet connection 18.

Furthermore, the device 12 comprises a nebulizer 26, arranged in a respective seat 28 made on the separator 20, and connected via a duct 30 to the inlet connection 16, so as to provide a fluid connection between the inlet connection 16 and the outlet connection 18, via a through hole 32 thereof, which has a Venturi tube shape comprising a narrowed section 36.

The first chamber 22 is in fluid connection with the second chamber 24 via at least one injector 34 provided on the nebulizer 26, at the narrowed section 36 and/or in a portion of the through-hole 32 downstream from said narrowed section 36.

According to a possible embodiment, the injectors 34 may be for example three in number and arranged equidistant along the circumference of the nebulizer 26.

Advantageously, the hole diameter of the at least one injector 34 may be between 0.1 mm and 0.25 mm.

For example, if the humidifier device 12 is used in a self-contained breathing apparatus, the inlet connection 16 will be used to fit the device to a pressure reducer (first stage), while the outlet connection 18 will be fit to the tube leading to the air dispenser (mouthpiece).

As may be seen in Figure 1, according to a possible embodiment, the body 14 may comprise two caps 142 and 144 of substantially cylindrical shape. The two caps 142, 144 may be joined together for example by means of a threaded connection provided at the ends of the caps themselves, as may be seen in the example of Figure 1. However, other embodiments of the connection between the caps 142, 144 are possible, such as for example an interlocking or bayonet connection.

Advantageously, a body gasket 143 may be arranged between the two caps 142, 144. The body gasket 143 may be, for example, an O-ring.

The body 14 may be provided with a cap 146 for filling the humidifying liquid 38.

According to a possible embodiment, the cap 146 may be provided at the cap 142 on which the inlet connection 16 is provided.

Advantageously, the connection between the stopper 146 and the body 14 may be of the threaded type, also by the interposition of a stopper gasket between the stopper 146 and the body 14. As may be seen in the example of Figure 1, the cap head may comprise a shaped impression 148 which may be engaged by a tool (not shown) to unscrew the cap 146.

Inside the first chamber 22, a non-return valve 42 is provided on the duct 30, adapted to allow the passage of air coming from the duct 30 to the first chamber 22, and to prevent the passage of fluid from the first chamber 22 to the duct 30. In particular, the valve 42 may be a valve of a difference of pressure type. The operation of the non-return valve 42 will be detailed below with particular reference to the operation of the humidifier device 12.

According to a possible embodiment, the valve 42 may be a valve comprising a ball 422 and a spring 424, arranged in such a way that the pressure difference between the duct 30 and the first chamber 22 causes the ball 422 to loosen from the respective seat, allowing the passage of air from the duct to the first chamber. However, the ball 422 moves against the action of the spring 424. Once the pressure equilibrium between the duct 30 and the first chamber 22 has been reached, the ball 422 returns to its seat under the action of the spring 424, closing the passage of air between the duct 30 and the first chamber 22. When the ball is in the respective seat, no fluid communication is possible from the first chamber 22 to the duct 30.

According to a possible embodiment, the duct 30 and the separator 20 may be a single component. Advantageously, in this case, the duct may be screwed into an inner portion of the inlet connection 16.

According to a possible embodiment, the separator 20 is provided with at least one channel 44 for the supply of liquid from the first chamber 22 to the nebulizer 26.

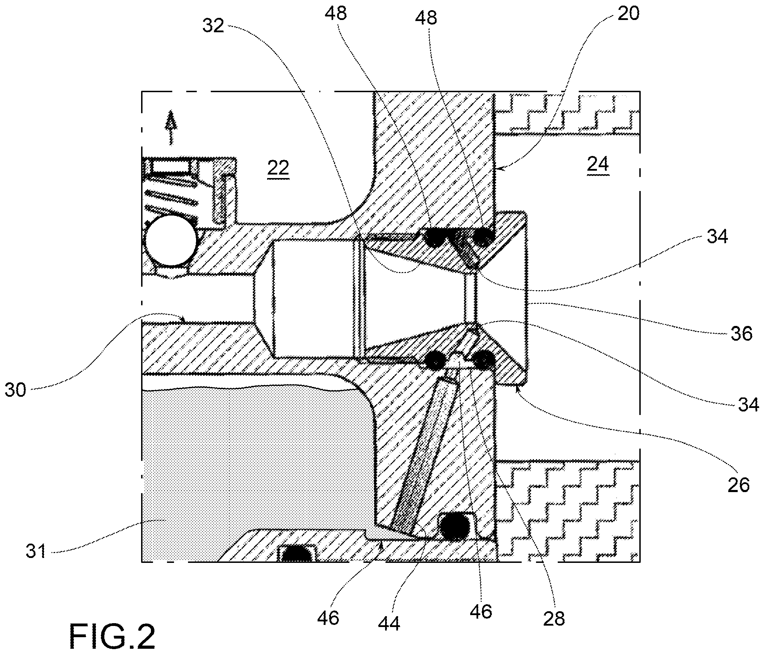

As may be seen in Figure 2, according to a possible embodiment, a radial slot 46 in fluid communication with the at least one channel 44 may be provided between the separator 20 and the body 14.

According to a possible embodiment, an annular channel 46 may be formed between the nebulizer 26 and the separator 20 for the supply of liquid to the at least one injector 34. Advantageously, the annular channel 46 may be made by means of an annular recess made on the outer surface of the nebulizer 26. In this case, the annular channel 46 may be limited longitudinally with two nebulizer seals 48 provided between the separator 20 and the nebulizer 26 itself.

As may be seen in Figure 2, the dry air coming from the duct 30, of which the nebulizer 26 may be substantially a continuation, reaches the nebulizer 26 and the liquid coming from the first chamber 22 passes through the at least one injector 34.

The nebulizer is therefore adapted for carrying out an air/liquid mixing, in order to obtain the nebulization of the liquid using the Venturi principle.

In fact, the air flowing into the duct 30 and subsequently into the nebulizer 26 passes through the narrowed section 36. At the narrowed section 36, the air undergoes an acceleration, thereby decreasing the pressure. By virtue of the decrease in pressure, there is a recall of the liquid through the at least one injector 34, which is nebulized in the flow of air flowing through the nebulizer 26.

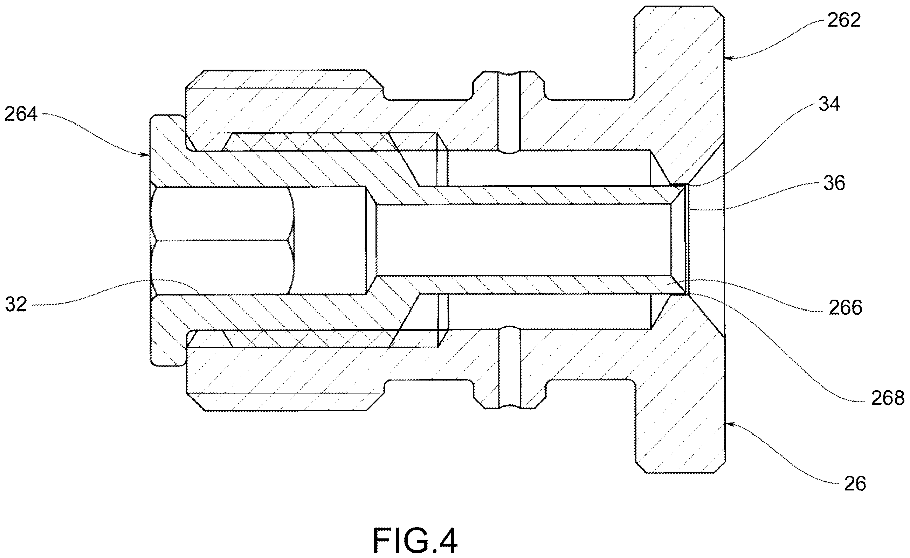

According to a possible embodiment, shown in Figure 4, the nebulizer 26 may be of the adjustable type, so that the effective supply of liquid substance to the air flow may be regulated.

The nebulizer 26 may comprise an external element 262 and an internal element 264, wherein the internal element 264 may be screwed onto the external element 262 so as to vary its axial position.

The internal element 264 comprises an elongated portion 266 adapted to couple with the narrowed section 36 to form a radial fluid meatus 268. The radial fluid meatus 268 corresponds to the injector 34. To this end, the injector 34 may be a circumferential meatus made between the narrowed section 36 and the elongated portion 266.

The adjustment of the injector 34 is carried out by screwing the internal element more or less to the external element so as to vary the axial length of the radial fluid meatus 268.

A drop collecting pad 50 may be provided inside the second chamber 24.

According to a possible embodiment, the drop collection pad 50 may have a substantially cylindrical shape with a longitudinal through hole 52, through which the humidified air passes.

Advantageously, the drop collection pad 50 may be made of antibacterial absorbent material and free from the formation of mold.

The operation of the humidifier device 12 of dry gaseous fluid according to the present invention will be described below.

With the actuation of the supply valve of the cylinder, the inlet air passes through the pressure reducer (first stage) in which it undergoes a first pressure reduction and is introduced into the device 12, passes through it, and using the appropriate ducts occupies all available spaces reaching the mouthpiece.

More precisely, the air enters the device 12, passes through the ducts available, occupies the available spaces and, passing through the non-return valve 42, enters the first chamber 22 (compensation chamber).

Specifically, in the embodiment which provides for a valve 42 provided with a ball 422, the air pressure causes the ball 422 to rise from its seat, thus allowing the access of air into the first chamber 22.

When the operating pressure in the first chamber 22 is reached and when the same has been balanced, the ball 422 is repositioned in its seat, which in this position prevents the outflow of air. The air introduced into the first chamber 22 will exert the pressure action on the free surface of the humidifying liquid 38.

The operator's breathing action causes a reduction in pressure in the second chamber 24 (nebulization chamber) and consequently a return of air from the first stage reducer. The following situation arises in this way:

a) formation of a depression in the second chamber 24;

b) generation of a greater pressure in the first chamber 22, which, acting on the surface of the humidifying liquid, presses it, forcing it to travel through the channel 44 which leads it to the at least one injector 34 of the nebulizer 26;

c) in the nebulizer 26 the humidifying liquid is brought into contact with the air, and as it passes through the small holes of the at least one injector it is reduced into microscopic particles which, by virtue of the Venturi effect, nebulize.

The advantages that may be achieved with a device according to the present invention are now apparent.

In the first place, the possible drawbacks caused by the assimilation of dry air, possibly by adding specific additives in addition to humidity, have been eliminated.

Secondly, the humidifier device is made without electrical and/or electronic and/or mechanical devices which, in the event of a malfunction, may affect the operation thereof. In fact, advantageously, the device may be made with simple and easily removable components, so that the inside of the device may be easily accessible and easily undergo maintenance.

Furthermore, all components may be made with materials that are not subject to oxidation or that do not allow the formation of mold.

The device is used in all industrial, sports and medical sectors.

Furthermore, the device may also be used for air humidification in small and large rooms where there is a need for air with controlled humidity.

The device may also be used in self-contained breathing apparatus for cleaning silos, cleaning tanks, maintenance of galvanic systems, etc., or in rescue (interventions on fires, environmental pollution by chemical substances, etc.). The device 12 may therefore be used in activities that require the use of a self-contained breathing apparatus possibly with the administration of drugs in aerosol, even in an emergency by introducing the drug through a specific one-way valve.

The device may be made of metallic material, or polymeric material.

A person skilled in the art will be able to make modifications to the embodiments described above and/or substitute described elements with equivalent elements in order to satisfy particular requirements, without departing from the scope of the accompanying claims.

## Claims

1. Humidifier device (12) of dry gaseous fluid comprising a body (14) with an inlet connection (16) and an outlet connection (18);
inside said body (14) being provided a separator (20) adapted to divide the inner volume of said body into two chambers (22, 24): a first compensation chamber (22), adapted to contain a liquid, provided near said inlet connection (16); and a second expansion and nebulization chamber (24) provided near said outlet connection (18); wherein said device (12) comprises a nebulizer (26) provided in a respective seat (28) made on said separator (20) ;
wherein said nebulizer (26) is connected via a duct (30) to said inlet connection (16), in order to realize a fluid connection between inlet connection (16) and outlet connection (18), via a through hole (32) thereof;
said first chamber (22) being in fluid connection with said second chamber (24) via at least one injector (34) provided in said nebulizer (26);
wherein said through-hole (32) of said nebulizer (26) has a venturi tube shape comprising a narrowed section (36), said at least one injector being provided at said narrowed section (36) and/or in a portion of the through-hole (32) downstream of said narrowed section (36).

2. Device (12) according to the preceding claim, wherein said injectors (34) are three in number and arranged equidistant along the circumference of the nebulizer (26).

3. Device (12) according to any of the preceding claims, wherein said body (14) comprises two caps (142, 144) of substantially cylindrical shape, joined together by a threaded connection arranged at the ends of said caps (142, 144).

4. Device (12) according to any of the preceding claims, wherein said body (14) is provided with a stopper (146) for the loading of the humidifying liquid (38) inside the first chamber (22); between said stopper (146) and said body (14) a threaded connection being provided, through the interposition, between stopper (146) and body (14) of a stopper gasket.

5. Device (12) according to any of the preceding claims, wherein there is a non-return valve (42) on the duct (30), adapted to allow the passage of air coming from the duct (30) to the first chamber (22), and to prevent the passage of fluid from the first chamber (22) to the duct (30), said valve (42) being a valve of a difference of pressure type.

6. Device (12) according to any of the preceding claims, wherein said separator (20) is provided with at least one channel (44) for the supply of liquid from the first chamber (22) to the nebulizer (26).

7. Device (12) according to any of the preceding claims, wherein between the nebulizer (26) and separator (20) there is an annular channel (46) for the supply of liquid to at least one injector (34).

8. Device (12) according to any of the preceding claims, wherein said nebulizer (26) comprises an external element (262) and an internal element (264), wherein the internal element (264) is screwed onto the external element (262) so as to vary its axial position; said internal element (264) comprising an elongated portion (266) adapted to couple with the narrowed section (36) to make a radial fluid meatus (268), said radial fluid meatus (268) corresponding to the injector (34), the adjustment of the injector (34) taking place by screwing the internal element more or less to the external element so as to vary the axial length of the radial fluid meatus (268).

9. Device (12) according to any of the preceding claims, wherein said second chamber (24) is provided with a drop collecting pad (50) of substantially cylindrical shape, with a longitudinal through hole (52), through which humidified air passes.

10. Device according to any one of the preceding claims, wherein said body (14), said separator (20) and said nebulizer (26) are made of metal and/or polymeric material.

## Patentansprüche

1. Befeuchtungsvorrichtung (12) für ein trockenes gasförmiges Fluid, die einen Körper (14) mit einem Einlassanschluss (16) und einem Auslassanschluss (18) umfasst,
wobei im Inneren des Körpers (14) ein Separator (20) vorgesehen ist, der geeignet ist, das Innenvolumen des Körpers in zwei Kammern (22, 24) zu unterteilen: eine erste Kompensationskammer (22), die geeignet ist, eine Flüssigkeit zu enthalten, und die in der Nähe des Einlassanschlusses (16) vorgesehen ist; und eine zweite Expansions- und Zerstäubungskammer (24), die in der Nähe des Auslassanschlusses (18) vorgesehen ist;
wobei die Vorrichtung (12) einen Zerstäuber (26) umfasst, der in einem entsprechenden Sitz (28) vorgesehen ist, der auf dem Separator (20) ausgebildet ist;
wobei der Zerstäuber (26) über eine Leitung (30) mit dem Einlassanschluss (16) verbunden ist, um eine Fluidverbindung zwischen dem Einlassanschluss (16) und dem Auslassanschluss (18) über ein Durchgangsloch (32) davon zu realisieren; wobei die erste Kammer (22) mit der zweiten Kammer (24) über mindestens einen Injektor (34), der in dem Zerstäuber (26) vorgesehen ist, in Fluidverbindung steht;
wobei das Durchgangsloch (32) des Zerstäubers (26) eine Venturi-Röhrenform aufweist, die einen verengten Abschnitt (36) umfasst, wobei der mindestens eine Injektor an dem verengten Abschnitt (36) und/oder in einem Teil des Durchgangslochs (32) stromabwärts des verengten Abschnitts (36) vorgesehen ist.

2. Vorrichtung (12) nach dem vorhergehenden Anspruch, wobei die Injektoren (34) drei an der Zahl und entlang des Umfangs des Zerstäubers (26) äquidistant angeordnet sind.

3. Vorrichtung (12) nach einem der vorhergehenden Ansprüche, wobei der Körper (14) zwei Kappen (142, 144) von im Wesentlichen zylindrischer Form umfasst, die durch eine an den Enden der Kappen (142, 144) angeordnete Gewindeverbindung miteinander verbunden sind.

4. Vorrichtung (12) nach einem der vorhergehenden Ansprüche, wobei der Körper (14) mit einem Stopfen (146) zum Einfüllen der Befeuchtungsflüssigkeit (38) in die erste Kammer (22) versehen ist; wobei zwischen dem Stopfen (146) und dem Körper (14) eine Gewindeverbindung vorgesehen ist, indem zwischen dem Stopfen (146) und dem Körper (14) eine Stopfendichtung angeordnet ist.

5. Vorrichtung (12) nach einem der vorhergehenden Ansprüche, wobei ein Rückschlagventil (42) an der Leitung (30) vorhanden ist, das dazu geeignet ist, den Durchgang von Luft, die aus der Leitung (30) kommt, zu der ersten Kammer (22) zu ermöglichen und den Durchgang von Fluid aus der ersten Kammer (22) zu der Leitung (30) zu verhindern, wobei das Ventil (42) ein Druckdifferenzventil ist.

6. Vorrichtung (12) nach einem der vorhergehenden Ansprüche, wobei der Separator (20) mit mindestens einem Kanal (44) für die Zufuhr von Flüssigkeit aus der ersten Kammer (22) zum Zerstäuber (26) versehen ist.

7. Vorrichtung (12) nach einem der vorhergehenden Ansprüche, wobei zwischen dem Zerstäuber (26) und dem Separator (20) ein ringförmiger Kanal (46) für die Zufuhr von Flüssigkeit zu mindestens einem Injektor (34) vorhanden ist.

8. Vorrichtung (12) nach einem der vorhergehenden Ansprüche, wobei der Zerstäuber (26) ein äußeres Element (262) und ein inneres Element (264) umfasst, wobei das innere Element (264) auf das äußere Element (262) geschraubt ist, um seine axiale Position zu verändern; wobei das innere Element (264) einen länglichen Teil (266) aufweist, der mit dem verengten Abschnitt (36) koppelbar ist, um einen radialen Fluidkanal (268) zu bilden, wobei der radiale Fluidkanal (268) dem Injektor (34) entspricht und die Einstellung des Injektors (34) durch mehr oder weniger starkes Verschrauben des inneren Elements mit dem äußeren Element erfolgt, um die axiale Länge des radialen Fluidkanals (268) zu verändern.

9. Vorrichtung (12) nach einem der vorhergehenden Ansprüche, wobei die zweite Kammer (24) mit einem Tropfenauffangkissen (50) von im wesentlichen zylindrischer Form versehen ist, mit einem länglichen Durchgangsloch (52), durch das befeuchtete Luft hindurchgeht.

10. Vorrichtung nach einem der vorhergehenden Ansprüche, wobei der Körper (14), der Separator (20) und der Zerstäuber (26) aus Metall und/oder polymerem Material hergestellt sind.

## Revendications

1. Dispositif humidificateur (12) de fluide gazeux sec comprenant un corps (14) avec une connexion d'entrée (16) et une connexion de sortie (18) ;
un séparateur (20) étant prévu à l'intérieur dudit corps (14), adapté pour diviser le volume intérieur dudit corps en deux chambres (22, 24) : une première chambre de compensation (22), adaptée pour contenir un liquide, prévue à proximité de ladite connexion d'entrée (16) ; et une seconde chambre d'expansion et de nébulisation (24) prévue à proximité de ladite connexion de sortie (18) ;
dans lequel ledit dispositif (12) comprend un nébuliseur (26) prévu dans un siège respectif (28) réalisé sur ledit séparateur (20);
dans lequel ledit nébuliseur (26) est connecté par l'intermédiaire d'un conduit (30) à ladite connexion d'entrée (16), afin de réaliser une connexion fluidique entre la connexion d'entrée (16) et la connexion de sortie (18), par l'intermédiaire d'un trou traversant (32) de celui-ci ;
ladite première chambre (22) étant en communication fluidique avec ladite seconde chambre (24) via au moins un injecteur (34) prévu dans ledit nébuliseur (26) ;
dans lequel ledit trou traversant (32) dudit nébuliseur (26) a une forme de tube de venturi comprenant une section rétrécie (36), ledit au moins un injecteur étant prévu à ladite section rétrécie (36) et/ou dans une partie du trou traversant (32) en aval de ladite section rétrécie (36).

2. Dispositif (12) selon la revendication précédente, dans lequel lesdits injecteurs (34) sont au nombre de trois et agencés à distance égale le long de la circonférence du nébuliseur (26).

3. Dispositif (12) selon l'une quelconque des revendications précédentes, dans lequel ledit corps (14) comprend deux capuchons (142, 144) de forme sensiblement cylindrique, reliés l'un à l'autre par une connexion filetée agencée aux extrémités desdits capuchons (142, 144).

4. Dispositif (12) selon l'une quelconque des revendications précédentes, dans lequel ledit corps (14) est pourvu d'un bouchon (146) pour le chargement du liquide d'humidification (38) à l'intérieur de la première chambre (22), une connexion filetée étant prévue entre ledit bouchon (146) et ledit corps (14), via l'interposition entre le bouchon (146) et le corps (14) d'un joint de bouchon.

5. Dispositif (12) selon l'une quelconque des revendications précédentes, dans lequel il existe un clapet anti-retour (42) sur le conduit (30), adapté pour permettre le passage d'air provenant du conduit (30) vers la première chambre (22), et pour empêcher le passage de fluide de la première chambre (22) vers le conduit (30), ledit clapet (42) étant un clapet d'un type à différence de pression.

6. Dispositif (12) selon l'une quelconque des revendications précédentes, dans lequel ledit séparateur (20) est pourvu d'au moins un canal (44) pour l'alimentation en liquide depuis la première chambre (22) vers le nébuliseur (26).

7. Dispositif (12) selon l'une quelconque des revendications précédentes, dans lequel entre le nébuliseur (26) et le séparateur (20) se trouve un canal annulaire (46) pour l'alimentation en liquide d'au moins un injecteur (34).

8. Dispositif (12) selon l'une quelconque des revendications précédentes, dans lequel ledit nébuliseur (26) comprend un élément externe (262) et un élément interne (264), dans lequel l'élément interne (264) est vissé sur l'élément externe (262) de manière à modifier sa position axiale ; ledit élément interne (264) comprenant une partie allongée (266) adaptée pour un couplage avec la section rétrécie (36) pour former un orifice de fluide radial (268), ledit orifice de fluide radial (268) correspondant à l'injecteur (34), l'ajustement de l'injecteur (34) ayant lieu en vissant l'élément interne plus ou moins sur l'élément externe de manière à modifier la longueur axiale de l'orifice de fluide radial (268).

9. Dispositif (12) selon l'une quelconque des revendications précédentes, dans lequel ladite seconde chambre (24) est pourvue d'un tampon de collecte de gouttes (50) de forme sensiblement cylindrique, avec un trou traversant longitudinal (52), à travers lequel passe de l'air humidifié.

10. Dispositif selon l'une quelconque des revendications précédentes, dans lequel ledit corps (14), ledit séparateur (20) et ledit nébuliseur (26) sont constitués de métal et/ou de matériau polymère.
